# EUROPEAN PATENT APPLICATION

(11) **EP 0 523 372 A1**
(43) Date of publication of application: **20.01.1993**
(21) Application number: 92109827.3
(22) Date of filing: 11.06.1992
(51) Int. Cl.: A61F 2/30, B22F 7/00, B22F 1/02, C23C 4/04

(54) **Medical material and process for its preparation**

(30) Priority: 12.06.1991 JP 167639/91
(71) Applicant: Nara Machinery Co., Ltd., Ohta-ku Tokyo (JP)
(72) Inventor: Oki, Sachio, Ashiya, Hyogo Pref. (JP); Gohda, Susumu, Sakai, Osaka Pref. (JP); Kimura, Tetsuo, Sagamihara, Kanagawa Pref. (JP); Ono, Kenji, Tokyo (JP)
(74) Representative: von Hellfeld, Axel, Dr. Dipl.-Phys.

(57) **Abstract**

Medical materials which comprise a substrate of a material having high strength and high biostability and a layer formed thereon by fusingly binding, while letting vacant pores be present, compounded particles prepared by subjecting core particles made of a material having high strength and high biostability to coating and fixing with a substance exhibiting high bioaffinity, and a process for preparing such medical materials, which comprises providing powdery compounded particles for use for thermal spraying by coating core particles made of a material having high strength and high biostability with a substance exhibiting high bioaffinity, and thermal spraying a substrate of the medical material made of a material having high strength and high biostability with said powdery compounded particles.

## Description

### FIELD OF THE INVENTION AND RELATED ART STATEMENT

The present invention relates to medical materials for supplementing deficient portions of hard tissue in organisms and to a process for their preparation. More particularly, the invention relates to medical materials for artificial bones for use in restorative surgery, artificial joints (portions connecting to living bones), artificial dental roots and so on, as well as to a process for their preparation.

In the field of medical treatment, there are employed metals, ceramics, high polymeric substances and various composite materials composed thereof (represented hereinafter inclusively as "implantation material") for supplementing deficient parts of various hard tissues in organisms which are selectively used in accordance with each specific deficient part. For such implantation materials, the safety and affinity to the organism is strictly required, in contrast to general industrial materials. It is also required to increase the performance for bonding to the living bone.

While metallic materials are superior in the strength and workability, their use is restricted by the possible poisoning to the organism and corrosion resistance. For example, when titanium (hereinafter represented by Ti) which has high strength and high biological stability is used as an implantation material, the bonding strength of the implantation material with living bones can be increased by making the surface thereof porous by subjecting it to flame spraying with powdery titanium metal over its surface, due to the increase in the contacting surface area, and in addition, the newly proliferated bone cells intrude into the pore spaces to firmly consolidate the implanted material. On the other hand, however, this also increases the dissolution of the material in the living organism due to an increase in the interfacial area with the organism. Even if a material, such as Ti mentioned above, has no toxicity to living organism when it dissolves to a certain extent, its progressive dissolution may cause the deterioration of its performance when implanted, so that any dissolution of the material should be suppressed as far as possible.

It is also known that the primary fixation of an implanted material to the living bone can be facilitated by coating the implantation material with a biophilic material having high affinity to living organism, such as an apatite, which is a principal constituent compound of the inorganic component of hard tissues in organisms, such as bones, teeth and so on. However, when a Ti implantation material covered by a porous surface layer which is formed by thermal spraying powdery Ti is subjected to direct thermal spraying over its surface with a fine particulate apatite, the porous surface structure becomes covered by the apatite and the effectiveness thereof is deteriorated. Moreover, it has not been possible to coat the internal pore surface of Ti with an apatite.

### OBJECT AND SUMMARY OF THE INVENTION

In consideration of the existing problems discussed above, an object of the present invention is to provide medical materials exhibiting high performance for bonding with living bones and capable of promoting the initial fixation onto the bones, and provide a process for preparing such medical materials. In addition to the above object, it is another object of the present invention is to provide medical implantation materials which suppress the dissolution of the substrate of the implantation material in living organisms and which thus prevent the deterioration thereof, and provide a process for preparing such implantation materials.

According to the present invention, the existing problems have been solved by medical materials which comprise a substrate of a material having high strength and high biostability and a layer formed thereon by fusingly binding, while letting vacant pores be present in the layer, powdery compounded particles prepared by subjecting core particles made of a material having high strength and high biostability to coating and fixing with a substance exhibiting a high bioaffinity.

Furthermore, the existing problems have also been solved according to the present invention by a process for preparing medical materials which comprises providing powdery compounded particles for plasma thermal spraying by coating core particles made of a material having high strength and high biostability with a substance exhibiting high bioaffinity, and plasma thermal spraying said powdery compounded particles onto a substrate made of a material having high strength and high biostability.

### BRIEF DESCRIPTION OF THE DRAWINGS

Fig. 1 is a photograph of particle structure of the surface of a compounded particle for plasma spraying according to the present invention by a scanning electron microscope.

Fig. 2 is an X-ray photograph of Ti on a surface of the same compounded particle of Fig. 1 taken using an X-ray microanalyzer.

Fig. 3 is an X-ray photograph of Ca on a surface of the same compounded particle corresponding to Fig. 2.

Fig. 4 is an X-ray photograph of P on a surface of the same compounded particle corresponding to Fig. 2.

Fig. 5 shows X-ray diffraction charts, in which (a) is the chart for a chemical reagent grade apatite, (b) for the compounded particles A for use for plasma thermal spraying, and (c) for a cover layer formed by plasma thermal spraying the compounded particles onto a substrate.

Fig. 6 is a photograph of particle structure of the surface of the implantation material I according to the present invention by a scanning electron microscope.

Fig. 7 is a photograph of particle structure of the surface of the implantation material II by a scanning electron microscope.

Fig. 8 is an enlarged photograph of Fig. 6 of particle structure by scanning electron microscope.

Fig. 9 is an X-ray photograph of Ti that corresponds to Fig. 8.

Fig. 10 is also an X-ray photograph of Ca that corresponds to Fig. 8.

Fig. 11 is a photograph of particle structure in a section of the plasma thermal sprayed cover layer of the implantation material I according to the present invention by a scanning electron microscope.

Fig. 12 is an X-ray photograph of Ti that corresponds to Fig. 11.

Fig. 13 is an X-ray photograph of Ca that corresponds to Fig. 11.

Fig. 14 is a drawing explaining how the powdery compounded particles A is prepared according to the present invention.

Fig. 15 is an explanatory diagram explaining the manner for forming a plasma thermal sprayed cover layer according to the present invention.

### DETAILED DESCRIPTION OF PREFERRED EMBODIMENTS

In preparing the medical material according to the present invention, powdery compounded particles (hereinafter referred to as a composite thermal spray powder) is first prepared, in which particles of a material having high strength and high bio-stability, such as the metal or ceramic mentioned previously, are coated with a material having high bioaffinity on their surfaces. For such preparation of the thermal spray powder, a surface modifying device for solid particles based on the high velocity gas stream impact method (see, for example, Japanese Patent Provisional Publication No. 83029/1987; referred to hereinafter simply as "surface modifying device") may be employed. This surface modifying device was developed for efficiently producing a functional composite particulate product in a dry system using a mechanical impact means with reduced production time, by fixing over the surface of a solid particle (referred to hereinafter as "mother particle") other solid particles of size smaller than said solid particle (hereinafter referred to as "child particles").

Here, for operating the surface modifying device, a rotor speed is preferably in a range of 50 to 100 m/sec in terms of the circumferential velocity. At a speed below 50 m/sec, a sufficient air flow will not be established in the gas circulation circuit, which causes an inefficient processing of the particulate product. On the other hand, with a rotor circumferential velocity of more than 100 m/sec, the proportion of crushed mother particles increases.

Then, a substrate made of a metal or a ceramic material having high strength and high biostability is plasma sprayed with the composite thermal spray powder obtained as above. For such plasma thermal spraying, a reduced pressure plasma spraying device is employed. The reduced pressure plasma spraying device used in the example of the present invention had a size of Φ 800 x 1,200 mm and was equipped with a water-cooled double-walled plasma spraying chamber for reduced pressure with a gas tightness of about 1 Pa in which a plasma torch PG-100 with an output level of 40 kW and a powder feed device PF-500-3 (both made by BAY STATE Co., Ltd.) were installed together with a driving mechanism for the substrate.

For the substrate of an implantation material and the mother particles of the composite thermal spraying powder according to the present invention, a metal, for example, a stainless steel (such as SUS 316, SUS 316L, SUS 317), a Co-Cr alloy (such as HS-21, HS-25, COP-1, and MP35N), Ti or a Ti-alloy (such as Ti-6Al-4V, a Ti-Ni alloy), tantalum or so on, or a ceramic material (such as alumina) may be used, while it should not be limited only to these examples and other materials may also be employed so long as they exhibit high strength and high biostability.

The average particle size may preferably be in the range of about 50-150 µm, while a size larger than this range may also be permitted. This is because it is said that pores having sizes of at least 150 µm have to be present over the surface of the substrate in order to permit newly proliferated bone cells to intrude into the internal pore vacancies of the implantation material, and also because it is difficult to obtain pores effective for the intrusion of the newly proliferated bone cells into the implantation material if the majority of the mother particles has a particle size of not more than 45 µm.

Also, it is not necessary that the substrate of the implantation material and the composite thermal spraying powder are made of the same material, but each should preferably be made of a material exhibiting a better fitting property with each other.

Furthermore, for the composite thermal spraying powder, for example, tricalcium phosphate which can be absorbed and replaced by the living bone tissue; hydroxylapatite and bioglasses which combine chemically with the living bone tissue; and so on can be employed, while it is not limited only to these materials but other substances having high bioaffinities may also be employed therefor. The particle size of the child particles of composite thermal spraying powder is smaller than that of the mother particles and should desirably be 1/5 at most, preferably 1/10 of the particle size of the mother particle. However, in the case of a material more brittle than the mother particles and if the material is subjected to crushing preferentially in the impact chamber, it is permissible that the particle size is comparable to that of the mother particles.

In preparing the composite thermal spraying powder, the proportion of the child particles necessary for surrounding or covering each mother particle can be determined beforehand, though roughly, by calculation based on the specific surface area of the mother particles, the particle size of the child particles and their true densities. However, so long as the surface of each mother particle is covered by the child particles at least nearly completely, the surface of the plasma sprayed layer and the inside pore surfaces of the implantation material become coated with the child particles when the substrate of the implantation material is finally plasma sprayed with the composite thermal spraying powder.

If the mother particles are crushed within the surface modifying device to form a fine dust or if the remainder of the child particles adhere on the circumference of the layer of child particles fixed on the surface of the mother particle or becomes present freely in the chamber, such fine particles may be removed by sieving before thermal spraying.

### Examples

### Preparation of Composite Thermal Spraying Powder

Fig. 14 conceptionally explains how the composite thermal spraying powder according to the present invention is prepared, in which a predetermined amount of the mother particles 10 and a predetermined amount of the child particles 12 are introduced into the surface modifying device 13 equipped with a rotor to effect processing under a specific condition to obtain the corresponding amount of the composite thermal spraying powder 14.

For the mother particles of the composite thermal spraying powder, a particulate product of pure Ti metal (TSP-100 manufactured by Osaka Titanium K.K., having a purity of over 99.5 % with particle sizes below 150 µm), and for the child particles, a commercial reagent of hydroxylapatite (by MERCK Co., Ltd., referred to hereinafter as "HAP") was employed respectively. The surface modifying device employed was NARA Hybridization System with a rotor of outer diameter of 118 mm (NHS-0 of NARA Machinery Co., Ltd.).

First, 50 g of a powder mixture composed of 45 g of the pure Ti particles and 5 g (10 % by weight) of HAP were introduced into the device, and thereby a composite thermal spraying powder A in which the fine particles of HAP were fixed onto the surface of each Ti particle was obtained. Here, the circumferential speed of the rotor was 80 m/sec, and the duration of the processing was 3 minutes.

Under the same processing condition, a composite thermal spraying powder B and a composite thermal spraying powder C having proportions of HAP particles of 20 % and 30 % by weight respectively were obtained.

### Evaluation of the Composite Thermal Spraying Powder

The surface and a section of a particle of each of the above composite thermal spraying powders were observed using a scanning electron microscope (abbreviated hereinafter as "SEM") and the particles were analyzed using an X-ray microanalyzer (JXA-8600M of JEOL Ltd.; abbreviated hereinafter as "EPMA"). By observing the surfaces, it was confirmed that the particle of composite thermal spraying powder A was a composite particle in which the surface of the Ti particle was almost completely covered by HAP particles fixed thereto. Similarly, the composite thermal spraying powder B was constituted of particles each composed of a Ti particle having a layer of HAP fine particles fixed thereto on which somewhat coagulated HAP particles are attached. The composite thermal spraying powder C had shown that particles of HAP remained freely. The average particle size of Ti was found to be slightly reduced by being crushed in the impact chamber of the surface modifying device. However, it was found that HAP particles were also fixed onto the Ti particles subjected to crushing.

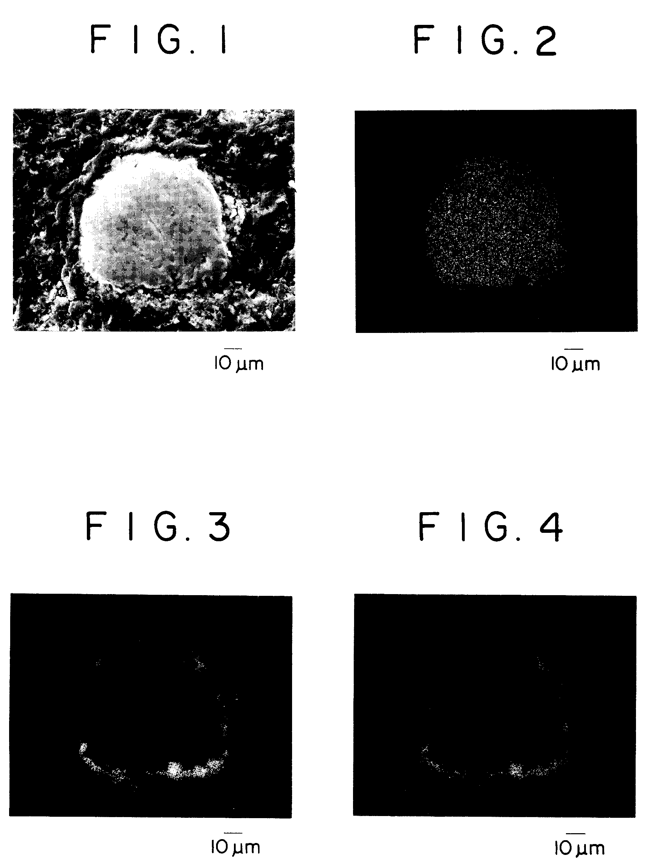

An SEM image of the surface of the composite thermal spraying powder A is shown in Fig. 1, and EPMA images of Ti as well as of Ca and P which are the principal component elements of HAP, which has a chemical formula of Ca₅(PO₄)₃(OH), are shown in Figs. 2 to 4, respectively. Thus, Fig. 2 is an X-ray photograph of Ti constituting the composite thermal spraying powder, Fig. 3 an X-ray photograph of Ca, and Fig. 4 an X-ray photograph of P. Also from these observations it is confirmed that the composite thermal spraying powder A was made of compounded particles in which each Ti mother particle was coated almost completely with HAP particles fixed thereto. Moreover, it was seen that the composite thermal spraying powder A was subjected during the compounding treatment to mechanical impacts by the rotor and to impingement onto the impingement ring, whereby the Ti particles were rubbed into spheres onto which HAP particles were fixed firmly.

### Formation of Plasma Sprayed Coating Layer

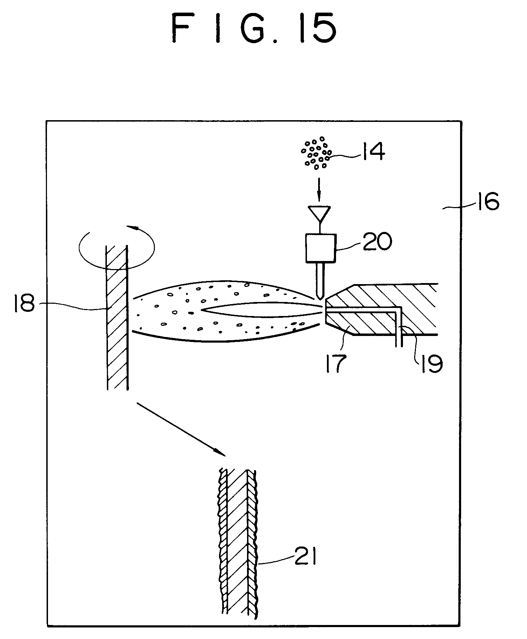

Fig. 15 conceptionally explains how the plasma sprayed coating layer is formed, in which 17 is a plasma spray nozzle, 18 is a substrate of an implantation material onto which the thermal spraying is effected and which is rotated by a driving means (not shown). Numeral 19 indicates a feed line for the carrier gas, such as Ar, to be discharged from the end of the thermal spraying nozzle 17, and 20 indicates a powder feed device for supplying the composite thermal spraying powder 14 prepared in the foregoing process step to the tip of the thermal spraying nozzle 17. These arrangements are installed within a sealable chamber 16. The plasma sprayed composite thermal spraying powder 14 will be fusingly fixed onto the surface of the substrate 18 to obtain the implantation material 21.

A pure Ti plate (KS-50 of Kobe Steel Ltd., having a purity over 99.5 %) cut into a size of 40 mm × 10 mm × 2 mm was used as the substrate 18, and this was mounted on the driving means, whereupon the internal space of the chamber is evacuated to a level of about 1 Pa, before Ar gas is introduced therein. Then, the composite thermal spraying powder A obtained previously was plasma sprayed under an environmental pressure of 13.3 kPa using Ar gas as the plasma gas at an electric voltage of 40 V and a current of 700 A at a plasma spray distance of 150 mm, whereby an implantation material I was obtained. The reason for effecting the thermal spraying in an atmosphere of Ar gas was that pure Ti would immediately be subjected to oxidation when it is subjected to thermal spraying in the air. The thermal spraying was effected to form a double layer by rotating the substrate, in order to obtain a plasma sprayed layer of a thickness of 200 to 300 µm. The surface of the substrate was treated by sand blasting with #24 alumina grit before the thermal spraying.

As a comparative example, an implantation material II was prepared using pure Ti plate by thermal spraying thereto only with the same pure Ti particles as those of the mother particles (TSP-100) in the above example under the same condition. Further, as another comparative example, an implantation material III was prepared using pure Ti plate by thermal spraying thereto with pure Ti particles having smaller particle size (TSP-350 of Osaka Titanium K.K., having a purity over 99.5 % with particle sizes below 45 µm) under the same condition.

### Evaluation of the Surface Roughness of the Coated Layer

### 1) Measurement of the Surface Roughness of the Coated Layer:

The surface roughness of each of the implantation materials obtained as above was determined (by SURFCORDER SE-300 of Komatsu Ltd.), and the results are summarized in Table 1. It is seen that both the average roughness and the maximum roughness for the implantation material I were considerably smaller than those for the implantation material II, despite of the fact that the same Ti particles as the implantation material II were employed for the mother particles, and the average and maximum roughnesses were comparable to those of implantation material III obtained by thermal spraying small size particles of TSP-350. This may be due to that the Ti particles are subjected to crushing within the surface modifying device to form a fine dust, as described previously. Thus, it is considered that, by effecting the thermal spraying after the removal of the fine dust by using, for example, a sieve of a mesh width of about 45 µm, a value comparable to that of the implantation material II may be attained.

However, it was found that the number of surface pores having opening widths of at least 150 µm and pore depths of at least 50 µm, into which surface pores the newly proliferated bone cells can possibly intrude, was 10 within the measuring range of 7 mm. This is less than the number of 17 for the implantation material II, but is quite high as compared with that of the implantation material III (which had only 2) and is believed to be sufficient for permitting intrusion of the newly proliferated bone cells.

**Table 1**

| Implantation Material | Average Roughness Ra (µm) | Maximum Roughness Rm (µm) |
|---|---|---|
| I (Example) | 9.90 | 74.8 |
| II (Comparative Ex.) | 16.60 | 133.9 |
| III (Comparative Ex.) | 9.87 | 76.1 |

### 2) Evaluation by X-ray Diffraction:

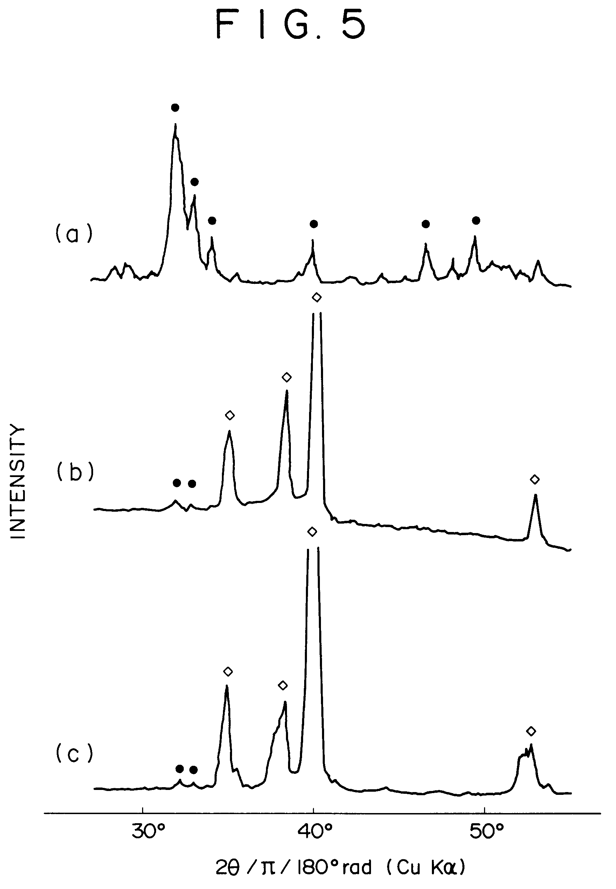

Existence of decomposition and reaction of HAP due to the preparation of the composite thermal spraying powder and due to the thermal spraying of such composite thermal spraying powder was examined by X-ray diffraction. The results are shown in Fig. 5. In this Figure, (a) is the X-ray diffraction chart for the starting reagent of the apatite, (b) for the composite thermal spraying powder A, and (c) for the surface of the plasma spray coated layer of the implantation material I, respectively. For the X-ray diffraction examination, a powder X-ray diffraction apparatus (Geigerflex RAD-rA of the firm Rigakudenki K.K.) was employed with CuKα rays monochromatized by a monochrometer at a tube voltage of 40 kV and a tube current of 80 mA.

Since the content of the fine HAP particles in the composite thermal spraying powder A is as low as 10 %, distinct peaks were not obtained. However, a weak indication of the peaks at 2ϑ = 32° and 33° (solid circles ● in the figure) corresponding to the diffractions on the lattice planes 121/210 and 300 of HAP was recognized. Similarly, a weak indication of peaks at the same positions was recognized also for the surface of plasma sprayed layer of the implantation material I. In all the cases, no decomposition of HAP was recognized. In Fig. 5, the mark ◇ indicates existence of Ti.

### 3) Observation by Electron Microscope:

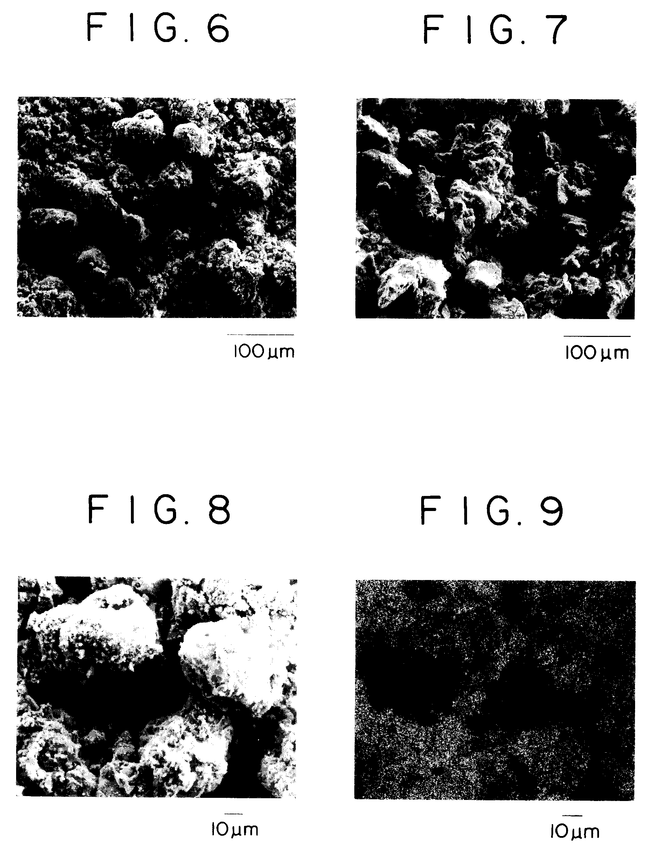

Fig. 6 shows an SEM image of the surface of the implantation material I obtained by thermal spraying the composite thermal spraying powder A, and Fig. 7 shows an SEM image of the surface of the implantation material II obtained by thermal spraying pure Ti particles (TSP-100).

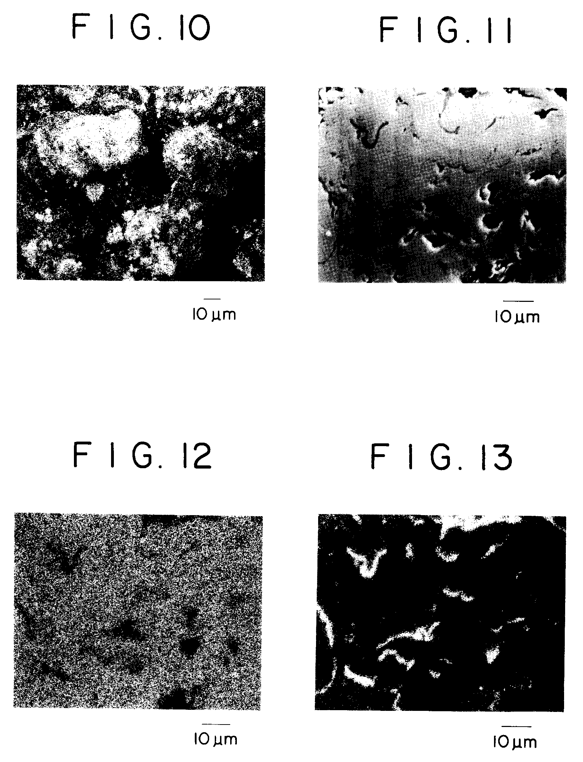

Figs. 8 to 10 are enlarged photographs of Fig. 6 showing an SEM image and EPMA images. Thus, Fig. 8 shows the SEM image, and Figs. 9 and 10 show an X-ray image of Ti and Ca, respectively. From these Figures, it is seen that the original shape of the particle of composite thermal spraying powder is inherited as such in the thermal sprayed cover layer, since the distribution of Ca (seen as white portions) is nearly uniform also in the thermal sprayed layer. Therefore, the HAP layer on the surface of the implantation material will, when it is embedded in a living body, accelerate the proliferation of new bone cells to increase the bonding strength, while suppressing dissolution of Ti, so that it is believed that deterioration of the implanted material can be prevented.

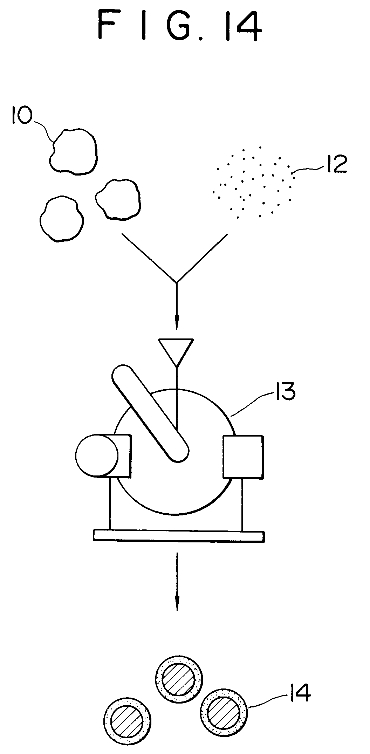

Figs. 11 to 13 show an SEM image and EPMA images of a section in the plasma sprayed layer of the implantation material I. Fig. 11 shows the SEM image and Figs. 12 and 13 show an X-ray image of Ti and Ca, respectively. From these pictures, it is seen that the HAP layer fixed onto the surface of Ti particle will not obstruct the fusing bonding of the Ti particles constituting the sprayed layer with each other, since it is recognized that Ca is distributed over the inside surface of the pores and over a part of the interfaces to the particles fusingly bonded and, at the same time, there are many interfaces between the Ti particles bonded directly together by fusing.

As described in detail above, by preparing a composite thermal spraying powder in which particles made of a material having high strength and high biostability, such as a metal or a ceramic having high strength and high biostability, are coated over their surfaces with a substance exhibiting high bioaffinity, and then causing this composite thermal spraying powder to be fixed fusingly onto a substrate made of high strength and high biostability, such as a metal or a ceramic having high strength and high biostability, by means of, for example, thermal spraying, the HAP layer on the surface of the resulting plasma sprayed cover layer facilitates proliferation of new bone cells, whereby it was possible to prepare implantation materials having high performance of combining with the living bone and permitting acceleration of primary bonding to the living bone.

It was also possible to prepare implantation materials in which the surface HAP layer on the plasma sprayed cover layer suppresses dissolution of Ti to thereby prevent deterioration of the implanted material.

Furthermore, it was possible to prepare implantation materials in which the fusion between a metal or ceramic material having high strength and biostability and such

## Claims

1. A medical material, comprising a substrate of a material having high strength and high biostability and a layer formed thereon by fusingly binding to the substrate, while letting vacant pores be present, compounded particles prepared by subjecting core particles made of a material having high strength and high biostability to coating and fixing with a substance exhibiting high bioaffinity.

2. A medical material as claimed in Claim 1, wherein said material having high strength and high biostability are selected from the group consisting of metals, ceramics and other substances exhibiting performances comparable to these.

3. A medical material as claimed in Claim 1, wherein said substance exhibiting high bioaffinity is selected from the group consisting of hydroxylapatite, tricalcium phosphate, bioglasses and other substances exhibiting performances comparable to these.

4. A process for preparing medical materials, comprising providing powdery compounded particles for plasma thermal spraying by coating core particles made of a material having high strength and high biostability with a substance exhibiting high bioaffinity, and plasma thermal spraying said powdery compounded particles onto a substrate made of a material having high strength and high biostability.

5. A process for preparing medical materials as claimed in Claim 4, wherein said material having high strength and high biostability is selected from the group consisting of metals, ceramics and other substances exhibiting performances comparable to these.

6. A process for preparing medical materials as claimed in Claim 4, wherein said substance exhibiting high bioaffinity is selected from the group consisting of hydroxylapatite, tricalcium phosphate, bioglasses and other substances exhibiting performances comparable to these.
